# EUROPEAN PATENT SPECIFICATION

(11) **EP 2 341 925 B1**
(45) Date of publication and mention of the grant of the patent: **26.11.2014**
(21) Application number: 09812761.6
(22) Date of filing: 20.08.2009
(51) Int. Cl.: A61K 35/56, A61K 38/48, A61P 7/02

(54) **THROMBOLYTIC/ ANTI THROMBOSIS AGENT AND ITS PRODUCTION METHOD**
THROMBOLYSEMITTEL/THROMBOSEHEMMER UND VERFAHREN ZU IHRER HERSTELLUNG
AGENT THROMBOLYTIQUE/ AGENT ANTI-THROMBOTIQUE ET SON PROCÉDÉ DE PRODUCTION

(30) Priority: 10.09.2008 ID 20080610
(43) Date of publication of application: 13.07.2011
(73) Proprietor: PT. Dexa Medica, Palembang, 30115 (ID)
(72) Inventor: TJANDRAWINATA, Raymond, R., Jakarta Selatan, 12310 (ID); TRISINA, Jessica, Jakarta Selatan, 12240 (ID)
(74) Representative: Petraz, Gilberto Luigi
(86) International application number: PCT/IB2009/053673
(87) International publication number: WO 2010/029453

(56) References cited:
- EP-A1- 2 105 139
- WO-A1-2006/034233
- WO-A1-2008/002019
- WO-A1-2008/013362
- WO-A1-2008/150375
- WO-A1-2009/001441
- WO-A1-2009/070818
- WO-A1-2009/078348
- WO-A2-2006/136540
- WO-A2-2007/002572
- WO-A2-2008/019417
- AU-A1- 2008 201 345
- CN-A- 1 229 852
- CN-A- 1 537 938
- CN-A- 1 651 079
- JP-A- 2004 292 402
- JP-A- 2006 096 673
- US-A- 4 568 545
- US-A- 5 024 844
- US-A- 5 186 944
- US-A1- 2008 206 352
- DATABASE WPI Week 200732 Thomson Scientific, London, GB; AN 2007-335844 XP002675732, & JP 2007 039404 A (INST BIOPHYSICS ACAD SINICA) 15 February 2007 (2007-02-15)
- DATABASE WPI Week 200873 Thomson Scientific, London, GB; AN 2008-M35595 XP002675733, & CN 101 229 369 A (HAERBIN SHENGYUAN BIOLOGICAL ENG CO LTD) 30 July 2008 (2008-07-30)
- PATENT ABSTRACTS OF JAPAN & JP 2 215 725 A (EIMEI:KK CO LTD) 28 August 1990
- PATENT ABSTRACTS OF JAPAN & JP 2 215 723 A (EIMEI:KK CO LTD) 28 August 1990
- PATENT ABSTRACTS OF JAPAN & JP 1 075 431 A (HISASHI, M.) 22 March 1989

## Description

### Field of the Invention

The present invention relates to a composition of thrombolytic agent and anti thrombosis which contains an earthworm extract that has thrombolytic and anti thrombosis activities, including combination of earthworm extract with other agents and pharmaceutical excipients. Production method of such earthworm extract include washing and freezing of the earthworm, extracting the frozen earthworm, separating the extract and its impurities, concentrating the extract and also granulating the earthworm extract. Composition of the present invention can be used as conventional medications or as alternative therapy to prevent or treat diseases related to thrombosis.

### Background of the Invention

In homeostasis system, blood clot or thrombus usually occurs in condition of injury or vascular damage to prevent bleeding; and its presence is regulated by the balance between fibrin formation and degradation pathways. During abnormal homeostasis condition, there is an imbalance between fibrin formation and degradation, causing abnormal thrombus formation which then leads to formation of thrombus-related pathologies such as stroke, coronary heart disease, lung embolism and deep vein thrombosis.

Current Fibrinolytic therapy uses fibrinolytic enzymes such as urokinase or plasminogen activator class (tPA and streptokinase). These enzymes have some limitations due to its relatively high price, its short half life and also its administration route via intravenous injection.

Earthworms have been known to have lumbrokinase enzyme, which belongs to a group of protease serine enzymes able to degrade fibrinogen and fibrin, resistant against high temperature, and can be absorbed via gastrointestinal tract, thus makes lumbrokinase a very potential agent used as an oral anti thrombosis - thrombolytic agent.

Ishii et al. stated that dry earthworm powder is useful as anti thrombosis drug (US Patent No. 5.186.944), anti hyperlipidemic, anti diabetic and blood pressure regulator (US Patent No. 5.128.148). The production process of the earthworm powder by such method is not practical. Earthworm immersing phase needs a special facility, long time and complicated standardization. Earthworm crushing phase needs special equipments. Drying technology using vacuum with increasing temperature gradually needs strict and complicated control; high temperature usage may inactivate the active component, lengthen the drying time and need a lot of energy. The resulting product will have a low purity level because there is no separation process between earthworm body parts and its active components.

Pharmaceutical compositions taught in the patents ('944 and '148) have degradation and reaction of the active Lumbrokinase component risk in gastric. Degradation may occur by physical degradation caused by peristaltic movement and chemical degradation by gastric acids and gastrointestinal enzymes in gastric. Lumbrokinase can react with protease-activated receptors (PARs/Protease Activated Receptors), which was taught in the patent no. WO/2008/013362 and Cho et al. (WO 2008/002019 A1), leading to a low absorption and a low bioavailability of lumbrokinase in blood; not reaching its effective dose

JP-A-2004292402 to Inukai and Mihara mentioned the method of manufacturing a dry powder earthworm. They taught the use of Alliaceae plant to remove the skin from the homogenized earthworms or from raw earthworms; as well as facilitate the removal of insoluble matter from the paste or extract.

Current therapeutic target from thrombus-related pathologies includes inhibition therapy against components in blood clotting pathway, such as thrombin inhibitor and blood clotting factor. Inhibition of platelet function and activation, plasminogen activation and also fibrin/thrombus degradation (fibrinolytic/thrombolytic).

### Brief Description of the Invention

The present invention aims to improve the weakness of current technology and to produce earthworm extracts that is useful to prevent and treat thrombosis. Composition which is produced from the present invention is stable, has efficacy as thrombolytic agent and anti thrombosis. In addition, pharmaceutical composition protects the active component from the damage and reaction in gastric.

This present invention also describes that the composition and production method of earthworm extract contains lumbrokinase as its active ingredient; the steps include: washing alive earthworm with water and alcohol, freezing earthworm, extracting lumbrokinase, separating dissolved and non dissolved extract components, concentrating, solidifying and drying the extract.

Composition of earthworm granules which is made by such method can be directly used as pharmaceutical dosage form or combined with suitable excipient(s) to produce pharmaceutical dosage form composition that ia useful to prevent and treat diseases related to vascular blockage because of thrombus.

### Brief Description of the Drawings

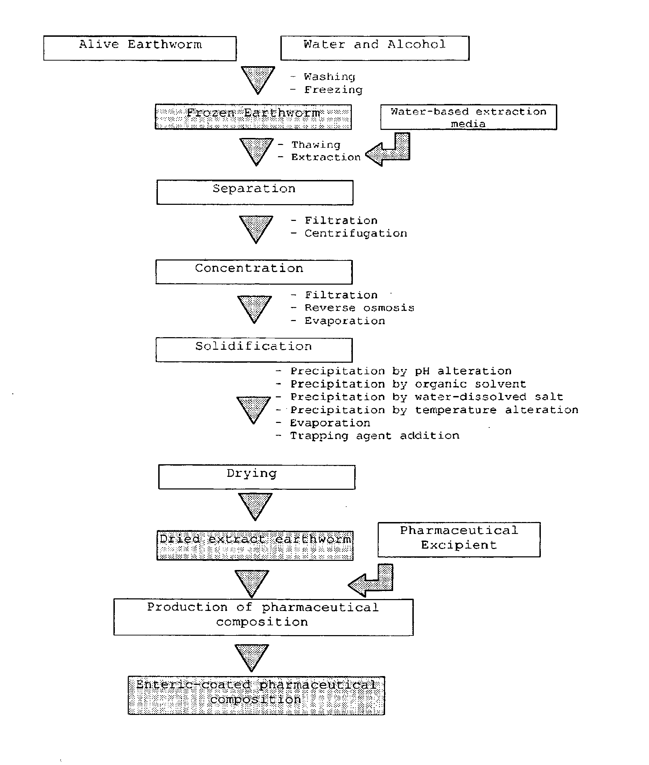

Figure No. 1 is a diagram of production method of earthworm extract according to the present invention.

### Detailed Description of the Invention

The invention will be made in detail by giving the examples without limiting the scope of the invention to the examples provided.

### A. Production of Earthworm Extract

The present invention aims to produce a composition of earthworm extract that has thrombolytic effect and anti thrombosis by simple and practical production process; therefore it can be easily applied in industrial scale. The production of this extract also eases the production process of oral dosage form composition, thus it can be directly used as pharmaceutical dosage form or combined with other suitable pharmaceutical excipients and made into enteric-coated form.

All kinds of earthworm can be used as raw materials in this earthworm extract production process, but preferably raw materials from Lumbricidae family, such as *Lumbricus rubellus* and *Eisenia foetida,* because it is easy and commonly cultivated, thus its continuity and homogeneity can be controlled.

Lumbrokinase, as active ingredient from earthworm that has thrombolytic and anti thrombosis activities, is present abundantly in coelomic liquid, blood, smooth muscle layer and also gut. Lumbrokinase is a serine protease that has an ability to degrade fibrinogen and fibrin, which are the key components in blood clotting pathway.

Fibrinogen is a glycoprotein which is a precursor in fibrin formation and plays an important role in various patophysiologycal processes, such as inflammation and thrombogenesis. Fibrinogen is one of determinant factors of blood viscosity, an inflammation-related acute phase protein. It also helps platelet aggregation process and is a substrate for thrombin in fibrin formation.

Plasma fibrinogen level is influenced by genetic and environment factors. Environment factors that are potential in increasing plasma fibrinogen level are aging, smoking habit, obesity, lacking of physical activity, increasing of cholesterol level, menopause and stress.

Increase of plasma fibrinogen level (hyperfibrinogemia) may cause prothrombotic condition that increases the risk of bloodvascular blockage (thromboembolism), cardiovascular impairment and also other related pathologies.

Fibrin is a major key of thrombus formation, where fibrin threads together with activated platelets will form thrombus nucleus or blood clot that can block blood vessel.

Due to its ability to degrade fibrinogen and fibrin, lumbrokinase derived from earthworm can be used as an agent that prevents thrombosis, as well as a thrombolytic agent. Lumbrokinase is absorbed through the intestine, therefore it can be administered orally.

The present invention describes a dried earthworm extract composition that is useful as an anti thrombosis agent and a thrombolytic agent, with lumbrokinase as its active ingredient, produced using a method that is practical and relatively simple to formulate into an oral solid pharmaceutical composition form.

The outer body of earthworm consists of skin layers semipermeable mucous membrane, muscle layer and smooth muscle layer on the inner side. Before it is used as raw material of the extract production, the earthworms should be cleaned from dirts attached on their body surfaces. Such dirts can be cleaned by washing it with clean water. After cleaning to remove dirts, the earthworms are then washed with alcohol. The earthworms washing with alcohol reduces the number of pathogenic microbes present on body surface and also make the surface body more permeable.

The earthworms are rinsed with water to wash away the remaining alcohol. The clean earthworms then are frozen at temperature between 0°C to -80°C, and preferably between 0°C to -20°C.

The extraction can be processed by using water-based media or by thawing.

The water-based extraction process uses maceration method with speed agitation. Water-based media that can be used are water, salt buffer, and mixture of water and organic solvent that is miscible with water. In salt buffer, cations that were used are from group I and II, while anions are from group VII and phosphate group. Ratio of earthworm mass and buffer volume that was used is 1:0.5-5, preferably 1:1-2. The extraction temperature is between 20°C-70°C, preferably 35°C-55°C, for 15 minutes to 5 hours, preferably 30 minutes to 2 hours.

In thawing extraction method, the extraction temperature is between 20°C-70°C, preferably 35°C-60°C. The extraction process includes agitation with low to moderate speed.

The extraction process by either of the above methods which utilizes the mentioned prepared earthworms as raw materials causes the earthworms to crumble by themselves; therefore crushing is not needed to prevent inactivation of active component(s) by mechanical, thermal and enzymatic degradation by its natural inhibitors.

Separation process between dissolved and non dissolved extract components is performed by mechanical separation. In this process, the lumbrokinase active component is in the dissolved phase thus it can be separated from other non dissolved components such as body parts of earthworms, dirts in gut, and other impurities.

The process can be performed by filtration, centrifugation or by its combination. Preferred process is filtration followed by centrifugation. Preferred filtration uses a filter with mesh size around 1.68-0.037 mm and preferably around 1-0.149 mm. It is also more preferable to perform in 2 steps: the first step is by mesh size around 1-0.42 mm and the second step is by mesh size around 0.149-0.105 mm. Centrifugation process is performed in speed around 1000-10000 g, preferably around 3500-7500 g. This separation process may separate the dirts in the earthworm gut from the extract without immersing process with acid solution, as taught by Ishii et al. in US Patent No. 5.186.944. This separation process also produces more pure extracts because it could separate other earthworm body parts.

Filtrate or supernatant produced is to be concentrated to 2-50% of the initial volume. Preferably, between 5-20% of the initial volume.The concentrating method utilizes a filtration system such as microfiltration, ultrafiltration, reverse osmosis, evaporation or its combinations. Preferred combination is reverse osmosis followed by evaporation. Temperature in concentrating condition is between 20-70°C.

The produced concentrate can be solidified or made into solid form by precipitation method using pH change, organic solvents, water soluble salt, temperature change, evaporation and addition of trapping agent.

Precipitation using pH change can be conducted by pH regulation between pH 2.5-6 and pH 8.5-13. Preferred pH is around 3-4.5 and pH 9-11. Precipitate which is formed, then will be centrifuged in order to separate the solid from the liquid.

Precipitation could use organic solvents that are miscible with water, such as alcohol and acetone group at 0-20°C, preferably using acetone at 0-5°C. Precipitate which is formed, then will be centrifuged in order to separate the solid from the liquid.

Precipitation could use water soluble salt, preferably ammonium sulphate in saturation level 20-80%. Preferred saturation level is 45-70%. Formed precipitate is to be centrifuged in order to separate the solid from the liquid.

Precipitation with temperature change is performed by cooling the concentrate at 0-10°C for 5-72 hours, preferably at 0-5°C for 8-12 hours.

Centrifugation process of precipitate obtained in the above method is conducted at speed of 1000-13000 g, preferably at speed of 5000-13000 g.

Evaporation process is conducted by using heat or freeze with freeze drying method.

Solidification process includes trapping agents and excipients. Trapping agents that are used is pharmaceutical fillers such as, microcrystalline cellulose, starch powder, lactose, sugar derivatives, calcium phosphate, etc, also silicon dioxide. Other materials are added in order to improve consistency of the solid produced or support final formulation, such as, binding agents: starch, gelatine, arabic gum, sugar derivatives, tragacanth, povidone, cellulose and its derivatives, sodium alginate, etc.; disintegrating agents: starch and its derivatives, primellose, primojel, etc. This process is preferred to solidify the concentrate.

The solid is dried out with suitable dryer such as oven, vacuum oven, Fluid Bed Dryer, Spray Dryer, Freeze Dryer or other commonly used dryer machines. Preferred drying method for solid with trapping process is by using Fluid Bed Dryer.

The production method of the extract described in the present invention has an advantage in short process time, therefore minimizes energy and cost used, and lowering contamination risk and product decomposition.

Dried earthworm extract can be used as a single component or combined with suitable pharmaceutical excipient(s) to form a pharmaceutical composition that can be used to prevent and treat thrombosis-related diseases.

Pharmaceutical composition that contains the earthworm extract can be used as preventive and treatment agents for diseases related to the thrombosis because it has fibrinogenolytic, fibrinolytic and thrombolytic activities.

Each step of the extract production process until final pharmaceutical composition can be tested with parameters related to the extract potency to include total protein content, detection and quantification of lumbrokinase through protease activity, fibrinogenolytic activity, fibrinolytic activity and also thrombolytic activity. Protease activity is evidenced by protease assay. Fibrinogenolytic activity is evidenced by fibrinogen substrate zymogram. Fibrinolytic activity is evidenced by fibrin plate method. Thrombolytic activity is proved by clot lysis assay.

The extract has a low microbe content and stable in closed storage at room temperature. Extract production steps are presented in Figure 1.

The following describes some examples that explain the present invention in more detail.

### Example 1

The fresh *Lumbricus rubellus* earthworms are washed with tap water until clean. The earthworms then are immersed in a basin contained 70% alcohol for 2 minutes. The earthworms are rinsed with purified water until alcohol smell disappears. The earthworms are packed with plastic and frozen at temperature 20°C.

### Example 2

The frozen *Lumbricus rubellus* from example 1 as much as 720 grams is extracted with 1980 mL potassium phosphate buffer 20 mM for 1 hour at 50°C. The suspension is centrifuged for 15 minutes, with the speed of 5000 rpm. The supernatant is then evaporated in vacuum condition at temperature 53°C until its volume reaches 9.3% of the initial volume. The concentrate is then mixed with 20% mass per volume corn starch and 15% mass per volume microcrystalline cellulose. Drying process is performed in vacuum oven for 3 hours at temperature 50°C. The extract granules produced are sieved using mesh no. 20.

### Example 3

The frozen earthworms from example 1 as much as 2900 grams are extracted with 7250 mL potassium phosphate buffer for 30 minutes. Suspension is filtered and then centrifuged at 5000 rpm, for 15 minutes. The supernatant is evaporated at temperature 55°C. The concentrate is mixed with 559.68 grams of microcrystalline cellulose 101; 262.35 grams of corn starch; 34.98 grams of sodium starch glycolate; and 17.49 grams of PVP-K30 with high shear mixer granulator. Wet granules are dried with Fluid Bed Dryer at drying temperature 50°C until the granules reach 5% water content.

### Example 4

As much as 826.6 grams of frozen earthworms are extracted using 1200 mL phosphate buffer for 30 minutes at temperature 50°C. Suspension is gone through staging filtration using mesh 30 and 100, and is centrifuged at 5000 rpm, 14 minutes, temperature 15°C. The supernatant is concentrated using reverse osmosis system. The volume of the concentrate reaches 30% of the initial volume.

### B. Pharmaceutical Dosage Forms

The present invention also includes composition and pharmaceutical dosage forms that contain earthworm extract in effective amount as an active ingredient in pharmaceutical dosage forms, including pharmaceutical carriers, excipients or additive substances that are pharmaceutically acceptable and physiologically suitable.

In pharmaceutical composition production process according to the present invention, the active ingredient of the earthworm extract is mixed with excipients, dissolved by excipients or mixed in pharmaceutical carriers that can be made in form of capsule, sachet, paper, also other materials or packaging. If the pharmaceutically acceptable excipient is used as solvent, such excipient can be in form of solid, semi-solid or liquid (oral and injection). It acts as a carrier or medium for the active ingredient. Therefore, pharmaceutical composition according to the present invention can be made in form of pill, capsule, tablet, powder, granule, sachet, solution, syrup, emulsion, suspension, effervescent tablet, gel, ointment, cream, and mouthwash, massage oil, suppository or injection. Beside that, pharmaceutical composition that contains earthworm extract according to the present invention can also be made as supplement, vitamin and also food and beverage production.

Some examples of excipients that are suitable such as microcrystalline cellulose, gelatine, lactose, dextrose, sucrose, sorbitol, mannitol, flour, calcium phosphate, calcium silicate, etc. Formulation according to the present invention may contain lubricant agents (such as talc, stearic magnesium, mineral oil), wetting agents, preservatives, sweetener and flavoring.

Composition according to the present invention can be made with the formulation which caused the active ingredient to release either directly, sustained or controlled after the patient received such composition, using methods which had been applied in pharmaceutical industry. Tablet or pill according to the present invention can be coated to extend the extract half life; thus its usage frequency can be reduced.

The composition production method in solid form, such as tablet, the active ingredient of earthworm extract can be mixed with excipients homogeneously according to the present invention. Such initial formulation is a mixture which contains active ingredient of earthworm extract that homogeneously dispersed and thus a dividing process can be conducted as required dose in form of granule, divided powder, pellet, capsule, pill, tablet and caplet. Such pharmaceutical composition can be made in its enteric-coated form to protect the active ingredient from degradation by acids, digestive enzymes and binding by protease receptors in gastric.

Excipient material that is being used in solid pharmaceutical composition includes fillers or diluent agents, such as: microcrystalline cellulose, starch powder, lactose, sugar derivatives, calcium phosphate, etc. Binding agents: starch, gelatine, arabic gum, sugar derivatives, tragacanth, povidone, cellulose and its derivatives, sodium alginate, etc. disintegrating agents: starch amd its derivatives, sodium starch glycolate, etc. Lubricant agents: stearic acid, stearic magnesium, stearic calcium, talc, silicon dioxide, etc. Enteric-coated agents: metacyrlate polymer, methyl cellulose and its derivatives, talc, titanium dioxide, coloring agent, propylene glycol and other materials that are commonly used in enteric-coated composition.

Solid pharmaceutical composition according to the present invention can be added with a protective layer to reduce or cover bitter taste from composition or active ingredient of earthworm extract.

Earthworm extract in concentration or effective dose according to the present invention is concentration or dose that the earthworm extract functions as thrombolytic agent and anti thrombosis. Effective dose depends on patient's physical condition, including bodyweight, age, etc.

Extract potency parameter tests until final pharmaceutical composition can be tested for its total protein content, detection and quantification of lumbrokinase through protease, fibrinogenolytic, fibrinolytic and also thrombolytic activities.

Earthworm extract and the composition which contains earthworm extract according to the present invention can be used effectively to prevent and treat diseases related to the thrombosis, such as stroke, coronary heart disease, lung embolism or deep vein thrombosis.

### C. Application in Industry

Extract and pharmaceutical dosage forms of this earthworms extract can be made in industrial scale in extract production, dry powder extract, and/or pharmaceutical dosage forms mainly for oral dosage form either solid, semi-solid or liquid in its usage as thrombolytic agent and anti thrombosis.

## Claims

1. A production method of earthworm extract comprising the steps of:
(a) preparation of earthworm raw materials by washing the alive earthworms with water and alcohol,
(b) freezing process of the earthworms,
(c) extraction process using water-based media using a maceration method with speed agitation or thawing process with agitating at low to moderate speed,
(d) separation process of the extract liquid with other earthworm body parts by filtration and/or centrifugation,
(e) concentrating process of the extract liquid,
(f) solidification process of the extract, and
(g) drying process of the earthworm extract.

2. The production method of earthworms extract according to claim 1, where alcohol concentration which is used in washing with alcohol in step (a) is a concentration of between 10% - 96%.

3. The production method of earthworms extract according to claim 1, where in step (b) freezing is performed at temperature of between -80°C to 0°C.

4. The production method of earthworms extract according to claim 1, where in step (c) the extraction process using water-based media, such as water, combination of water and organic solvent(s), and/or salt buffer.

5. The production method of earthworms extract according to claim 4, where the organic solvents are miscible with water, such as alcohol, acetone, or its combination(s).

6. The production method of earthworms extract according to claim 4, where salt is water-soluble salt.

7. The production method of earthworms extract according to claim 1, where in step (e), concentrating process is using a filtration system that is selected from a group comprising: microfiltration, ultrafiltration, reverse osmosis, evaporation or combination(s) thereof.

8. The production method of earthworms extract according to claim 1, where in step (f), solidification process of extract is using a process that is selected from a group comprising: precipitation method with pH change, organic solvent, water-soluble salt, temperature alteration, evaporation, and addition of trapping agent.

9. A pharmaceutical composition containing the extract prepared using the process according to claim 1 made in the form of enteric-coated solid dosage form for drug, supplement, vitamin, also food and beverage products.

## Patentansprüche

1. Ein Verfahren zur Herstellung von Regenwurmextrakt, umfassend die Schritte:
(a) Vorbereiten des Regenwurm-Rohmaterials durch Waschen der lebendigen Regenwürmer mit Wasser und Alkohol,
(b) Einfrierverfahren der Regenwürmer,
(c) Extraktionsverfahren unter Verwendung von wasserbasierten Medien unter Verwendung eines Mazerationsverfahrens mit schnellem Schütteln oder eines Auftauverfahrens mit langsamen bis moderatem Schütteln,
(d) Trennverfahren der extrahierten Flüssigkeit von anderen Regenwurm-Körperteilen mittels Filtrierung und/oder Zentrifugation,
(e) Konzentrierverfahren der extrahierten Flüssigkeit,
(f) Verfestigungsverfahren des Extrakts, und
(g) Trocknungsverfahren des Regenwurmextrakts.

2. Das Verfahren zur Herstellung von Regenwurmextrakt gemäß Anspruch 1, wobei die Alkoholkonzentration, die zum Waschen mit Alkohol in Schritt (a) verwendet wird, einer Konzentration von zwischen 10% und 96% ist.

3. Das Verfahren zur Herstellung von Regenwurmextrakt gemäß Anspruch 1, wobei in Schritt (b) das Einfrieren bei einer Temperatur von zwischen -80°C bis 0°C durchgeführt wird.

4. Das Verfahren zur Herstellung von Regenwurmextrakt gemäß Anspruch 1, wobei in Schritt (c) das Extraktionsverfahren wasserbasierte Medien, wie Wasser, eine Kombination aus Wasser und organischem/n Lösungsmittel(n) und/oder Salzpuffer verwendet.

5. Das Verfahren zur Herstellung von Regenwurmextrakt gemäß Anspruch 4, wobei die organischen Lösungsmittel mischbar mit Wasser sind, so wie Alkohol, Aceton oder (eine) Kombination(en) davon.

6. Das Verfahren zur Herstellung von Regenwurmextrakt gemäß Anspruch 4, wobei das Salz wasserlösliches Salz ist.

7. Das Verfahren zur Herstellung von Regenwurmextrakt gemäß Anspruch 1, wobei in Schritt (e) das Konzentrierverfahren ein Filtrationssystem verwendet, ausgewählt aus einer Gruppe umfassend: Mikrofiltration, Ultrafiltration, Umkehrosmose, Verdampfung oder (eine) Kombination(en) davon.

8. Das Verfahren zur Herstellung von Regenwurmextrakt gemäß Anspruch 1, wobei in Schritt (f) das Verfestigungsverfahren des Extrakts ein Verfahren verwendet, das ausgewählt wird aus einer Gruppe umfassend: Präzipitationsverfahren mit pH-Änderung, organisches Lösungsmittel, wasserlösliches Salz, Temperaturveränderung, Verdampfung und Zugabe eines Bindemittels.

9. Eine pharmazeutische Zusammensetzung, die den Extrakt enthält, der unter Verwendung des Verfahrens gemäß Anspruch 1 hergestellt wurde, hergestellt in einer magensaftresistenten festen Darreichungsform für Arzneimittel, Ergänzungsmittel, Vitamine sowie Nahrungsmittel- und Getränkeprodukte.

## Revendications

1. Procédé de production d'un extrait de vers de terre qui comprend les étapes consistant à :
(a) préparer les matières premières de vers de terre en lavant les vers de terre vivants avec de l'eau et de l'alcool,
(b) congeler les vers de terre,
(c) extraire à l'aide de milieux à base d'eau en utilisant un procédé de macération avec agitation à vitesse élevée ou de décongélation avec agitation à vitesse faible à modérée,
(d) séparer l'extrait liquide des autres parties du corps des vers de terre par filtration et/ou centrifugation,
(e) concentrer l'extrait liquide,
(f) solidifier l'extrait, et
(g) sécher l'extrait de vers de terre.

2. Procédé de production d'un extrait de vers de terre selon la revendication 1, dans lequel la concentration d'alcool utilisée lors du lavage à l'alcool à l'étape (a) est comprise entre 10 % et 96 %.

3. Procédé de production d'un extrait de vers de terre selon la revendication 1, dans lequel la congélation réalisée à l'étape (b) est effectuée à une température comprise entre -80°C et 0°C.

4. Procédé de production d'un extrait de vers de terre selon la revendication 1, dans lequel l'extraction réalisée à l'étape (c) est effectuée à l'aide de milieux à base d'eau, comme de l'eau, une combinaison d'eau et de solvant(s) organique(s) et/ou un tampon de sel.

5. Procédé de production d'un extrait de vers de terre selon la revendication 4, dans lequel les solvants organiques, comme l'alcool, l'acétone ou des combinaisons de ces derniers, sont miscibles à l'eau.

6. Procédé de production d'un extrait de vers de terre selon la revendication 4, dans lequel le sel est un sel soluble dans l'eau,

7. Procédé de production d'un extrait de vers de terre selon la revendication 1, dans lequel la concentration réalisée à l'étape (e) utilise un système de filtration sélectionné dans un groupe comprenant la microfiltration, l'ultrafiltration, l'osmose inverse, l'évaporation ou une/des combinaison(s) de ces derniers.

8. Procédé de production d'un extrait de vers de terre selon la revendication 1, dans lequel la solidification de l'extrait réalisée à l'étape (f) utilise un processus sélectionné dans un groupe comprenant un procédé de précipitation avec changement du pH, solvant organique, sel soluble dans l'eau, altération de température, évaporation et ajout d'agent de rétention.

9. Composition pharmaceutique contenant l'extrait préparé à l'aide du processus selon la revendication 1 sous forme dosée solide à enrobage entérique pour médicaments, compléments, vitamines ainsi que pour des produits alimentaires et des boissons.
